(19) Europäisches Patentamt
European Patent Office
Office européen des brevets

(11) **EP 0 709 411 B1**

(12) **EUROPÄISCHE PATENTSCHRIFT**

(45) Veröffentlichungstag und Bekanntmachung des Hinweises auf die Patenterteilung:
**17.06.1998  Patentblatt 1998/25**

(51) Int Cl.$^6$: **C08F 226/06**, A61K 7/06

(21) Anmeldenummer: **95116525.7**

(22) Anmeldetag: **20.10.1995**

(54) **Lösliche Copolymerisate für die Haarkosmetik**

Soluble copolymers for hair cosmetics

Copolymères solubles pour cosmétiques capillaires

(84) Benannte Vertragsstaaten:
**BE CH DE ES FR GB IT LI NL**

(30) Priorität: **29.10.1994  DE 4438706**

(43) Veröffentlichungstag der Anmeldung:
**01.05.1996  Patentblatt 1996/18**

(73) Patentinhaber: **BASF AKTIENGESELLSCHAFT
67056 Ludwigshafen (DE)**

(72) Erfinder:
* **Blankenburg, Rainer, Dr.
  D-67067 Ludwigshafen (DE)**
* **Sanner, Axel, Dr.
  D-67227 Frankenthal (DE)**

(56) Entgegenhaltungen:
**EP-A- 0 545 209          WO-A-92/07011**

## Beschreibung

Die vorliegende Erfindung betrifft Copolymerisate mit K-Werten von 30 bis 50 gemessen in 1 gew.-%iger ethanolischer Lösung bei 25°C, erhältlich durch radikalische Lösungspolymerisation von

A) 15 bis 84,99 Gew.-% mindestens eines Monomeren aus der Gruppe N-Vinylpyrrolidon, N-Vinylcaprolactam und N-Vinylimidazol,

B) 15 bis 84,99 Gew.-% weiterer radikalisch copolymerisierbarer monoolefinisch ungesättigter Monomeren und

C) 0,01 bis 2 Gew.-% mindestens eines radikalisch copolymerisierbaren Monomeren mit mindestens zwei nicht konjugierten olefinischen Doppelbindungen

in alkoholischer Lösung.

Weiterhin betrifft die Erfindung die Herstellung solcher Copolymerisate sowie deren Verwendung in der Haarkosmetik.

Als Filmbildner für die Haarkosmetik geeignete Polymere auf Basis von N-Vinylverbindungen und Acrylaten, welche durch radikalische Lösungspolymerisation in Alkoholen hergestellt werden, sind beispielsweise aus der DE-A-36 27 969, der DE-A-33 12 668, der DE-A-36 27 970 oder der DE-A-4 013 872 bekannt.

Aus der DE-A-28 14 287 sind Copolymere des N-Vinylimidazols bekannt, die weniger als 5 mol-% an Comonomeren mit zwei oder mehr copolymerisierbaren Doppelbindungen enthalten können, wobei solche Comonomeren zu dreidimensional vernetzten Polymeren führen. Die Herstellung dieser Copolymeren erfolgt in Wasser, Wasser-Methanol-Gemischen oder organischen Lösungsmitteln wie Toluol. Die Copolymeren eignen sich als verfärbungsinhibierende Additive in Waschmitteln.

Die Herstellung von als Filmbildner in der Haarkosmetik geeigneten Polymeren auf Basis von N-Vinylverbindungen und Acrylaten erfolgt üblicherweise durch radikalische Lösungspolymerisation in alkoholischen Lösungen, wobei man als Lösungsmittel vor allem die in der Kosmetik üblichen Lösungsmittel wie Ethanol und/oder Isopropanol, gegebenenfalls in Gemisch mit Wasser, verwendet.

Aufgrund der molekulargewichtsregelnden Eigenschaften dieser Alkohole, kann die gezielte Herstellung von Polymeren mit höheren K-Werten, beispielsweise im Bereich von 35-45, Schwierigkeiten bereiten. Höhere K-Werte lassen sich nach bekannten Verfahren nur bei geringerer Lösungsmittelkonzentration erzielen, was vor allem im Produktionsmaßstab Probleme bei der Wärmeabführung bereiten kann.

Andererseits weisen aber solche höhermolekularen Polymere bessere Festigungseigenschaften auf dem Haar auf.

Aufgabe der vorliegenden Erfindung war es für die Haarkosmetik geeignete höhermolekulare filmbildende Polymere zu finden, welche einfach durch radikalische Lösungspolymerisation in der Kosmetik üblichen Alkoholen zugänglich sind und in diesen Alkoholen gut löslich sind.

Demgemäß wurden die eingangs definierten Copolymere sowie ein Verfahren zu deren Herstellung, deren Verwendung und haarkosmetische Mittel, enthaltend diese Copolymere, gefunden.

Als Monomere A) kommen erfindungsgemäß heterocyclische N-Vinylverbindungen aus der Gruppe N-Vinylpyrrolidon, N-Vinylcaprolactam oder auch N-Vinylimidazol oder deren Gemische in Betracht. Bevorzugte Monomere A) sind N-Vinylpyrrolidon und N-Vinylcaprolactam.

Die Monomeren A) werden in Mengen von 15 bis 84,99, bevorzugt 20 bis 80 Gew.-% eingesetzt.

Als Monomere B) kommen erfindungsgemäß weitere monoolefinisch ungesättigte, radikalisch copolymerisierbare Verbindungen oder deren Gemische in Betracht, beispielsweise Vinylester von gesättigten $C_2$-$C_{24}$-Monocarbonsäuren wie Vinylacetat oder Vinylpropionat oder Vinylester längerkettiger Fettsäuren wie beispielsweise der Dodecansäure oder Stearinsäure, oder Vinylester verzweigter Fettsäuren wie Pivalinsäure oder Versaticsäure.

Weiterhin eignen sich $C_1$-$C_{10}$-Alkylester der Acrylsäure oder der Methacrylsäure, beispielsweise Ethylacrylat, Isobutylacrylat, n-Butylacrylat, tert.-Butylacrylat, Methylmethacrylat, Ethylmethacrylat, Isobutylmethacrylat oder n-Butylmethacrylat, oder auch die entsprechenden Ester von Aminoalkanolen wie beispielsweise N,N-Dimethylaminoethylmethacrylat.

Weiterhin eignen sich als Monomere B) auch sulfonatgruppenhaltige Monomere wie die Salze von 3-Acrylamido-2-methyl-propansulfonsäure oder 2-Acrylamido-2-methylpropansulfonsäure, bevorzugt die entsprechenden Natriumsalze.

Bevorzugte Monomere B) sind Vinylacetat und tert.-butylacrylat.

Die Monomeren B) wurden erfindungsgemäß in Mengen vcn 15 bis 84,99, bevorzugt 20 bis 80 Gew.-% eingesetzt.

Als Monomere C) kommen radikalisch copolymerisierbare Monomere in Betracht, die mindestens zwei nicht konjugierte olefinische Doppelbindungen im Molekül enthalten. Geeignete Monomere sind beispielsweise Acrylsäure- oder Methacrylsäureester von gesättigten mehrwertigen Alkoholen, beispielsweise 1,4-Butandioldiacrylat, 1,6-Hexandioldiacrylat, Tetraethylenglykoldiacrylat, Trimethylolpropantriacrylat, Tripropylenglykoldiacrylat, Pentaerythrittriacrylat, Pentaerythrittetraacrylat, Ethylenglykoldimethacrylat, Diethylenglykoldimethacrylat, Triethylenglykoldimethacrylat, Tetraethylenglykoldimethacrylat, Polyethylenglykol-400-dimethacrylat, 1,3-Butandioldimethacrylat, 1,4-Butandioldimethacrylat, 1,6-Hexandioldimethacrylat, 1,12-Dodecandioldimethacrylat, Neopentylglykoldimethacrylat oder Trimethylolpropantrimethacrylat.

Geeignete Monomere C) sind auch Allylmethacrylat oder BisphenolA-dimethacrylat, weiterhin Vinylether mehrwertiger Alkohole wie Butandioldivinylether. Ebenso eignen sich polyfunktionelle Acrylamide oder Methacrylamide wie beispielsweise N,N'-Methylen-bisacrylamid, N,N'-Methylen-bis-methacrylamid, N,N'-Oxibismethylenbis-acrylamid, N,N'-Benzyliden-bis-acrylamid, Terephthalidentetraacrylamid, N,N'-Butyliden-bis-acrylamid oder Bis-acrylamidoessigsäuremethylester. Weiterhin kommt als Monomer C) auch Divinylethylenharnstoff in Betracht.

Bevorzugte Monomere C) sind 1,4-Butandioldiacrylat, 1,6-Hexandioldiacrylat, Tetraethylenglykoldiacrylat, Ethylenglykoldimethacrylat, Butandioldivinylether, N,N'-Methylenbisacrylamid und Divinylethylenharnstoff.

Die Monomeren C) werden in Mengen von 0,01 bis 2 Gew.-%, bevorzugt 0,02 bis 0,5 Gew.-%, bezogen auf die gesamte Monomerenmischung, eingesetzt.

Bevorzugt wird die Menge an Monomeren C) so bemessen, daß der molare Anteil (N) an Monomeren C), bezogen auf die die Molzahl (P) eines Copolymerisats wie es ohne Verwendung des Monomeren c) entstehen würde, durch die folgende Beziehung

$$P/N = a$$

wobei a eine Dezimalzahl von 1 bis 5, bevorzugt 1,5 bis 3, bedeutet, charakterisiert ist. Die Größe (P) ist für den Fachmann leicht aus dem Verhältnis der Summe der Einwaagen an Monomeren A) und Monomeren B) zum mittleren Molekulargewicht $M_n$ eines Polymeren, wie es aus diesen Mengen an A) und B) erhalten wird, zu ermitteln.

Die erfindungsgemäßen Copolymerisate sind in kosmetischen Alkoholen wie Ethanol oder Isopropanol vollständig löslich und frei von Quellkörpern.

Die K-Werte betragen 30 bis 50, bevorzugt 35 bis 45. Die Bestimmung der K-Werte erfolgt nach "H. Fikentscher, CelluloseChemie, 13 (1932), S. 58-64 und S. 71-74 gemessen in 1 gew.-%iger ethanolischer Lösung bei 25°C.

Die Herstellung der erfindungsgemäßen Copolymerisate erfolgt nach der Methode der radikalischen Lösungspolymerisation in Alkoholen, gegebenenfalls in einem Gemisch aus Alkoholen und Wasser. Als Alkohole eignen sich $C_1$-$C_4$-Alkanole, wobei die in der Kosmetik üblicherweise verwendeten Alkohole Ethanol und Isopropanol bevorzugt sind.

Die Polymerisation erfolgt in Gegenwart geeigneter Mengen einer radikalbildenden Verbindung wie organischen Azo- oder Peroxo-Verbindungen als Polymerisationsinitiatoren.

Geeignete Initiatoren sind z.B. Diacylperoxide wie Dilauroyl-, Didecanoyl- und Dioctanoylperoxid, sowie Perester wie tert.-Butylperoxipivalat, tert.-Amylperoxipivalat oder tert.-Butylperoxineodecanoat, sowie Azoverbindungen wie Dimethyl-2,2-azobis(isobutyrat), 2,2-Azobis(isobutyronitril), 2,2-Azobis-(2methyl-butyronitril) oder 2,2-Azobis-(2,2-dimethylvaleronitril).

Die Herstellung der Copolymerisate kann im Batch-Verfahren erfolgen, bevorzugt jedoch nach dem Zulaufverfahren, wobei sich ein Teil der Lösung des Monomerengemischs, in der Vorlage befindet und der restliche Teil über einen Zeitraum von mehreren Stunden zugefahren wird. Die Monomeren C) werden bevorzugt nur über den Zulauf zugegeben.

Die Dauer des Monomerenzulaufs wird üblicherweise so bemessen, daß die freigesetzte Polymerisationswärme unter den üblichen technischen Bedingungen gut abgeführt werden kann.

Die Polymerisation wird vorteilhafterweise bei Temperaturen von 60 bis 100°C, vorzugsweise 70 bis 85°C durchgeführt, wobei die Reaktion unter Eigendruck, Normaldruck oder unter Schutzgasüberdruck durchgeführt werden kann. Als Schutzgas eignet sich beispielsweise Stickstoff.

Es kann sich auch empfehlen, im Anschluß an die Polymerisation eine Nachpolymerisation in Gegenwart weiterem Radikalinitiators durchzuführen. Als Radikalinitiatoren für die Nachpolymerisation eignen sich besonders Verbindungen der allgemeinen Formel I

$$R^1\text{-O-O-}R^2 \qquad\qquad I$$

worin $R^1$ für einen Alkylrest mit 4 bis 8 C-Atomen steht und $R^2$ für Wasserstoff oder $R^1$ oder für -$R^3$-O-O-$R^1$, worin $R^3$ einen linearen oder verzweigten $C_4$-$C_{10}$-Alkylenrest bedeutet. Als Alkylreste kommen beispielsweise tert.-Butyl- oder t-Amyl-Reste in Betracht. Hat $R^2$ die Bedeutung Wasserstoff, so handelt es sich um Alkylhydroperoxide. Ein geeigneter Radikalinitiator ist beispielsweise 2,5-Dimethyl-2,5-bis (tert.-butylperoxy)-hexan. Bevorzugte Radikalinitiatoren für die Nachpolymerisation sind Di-tert.butylperoxid und Di-tert.-amylperoxid. Die Nachpolymerisation wird vorteilhafterweise bei 110 bis 150°C solange durchgeführt, bis Restmonomerengehalte unter 50 ppm erreicht sind.

Die erfindungsgemäßen Copolymerisate weisen K-Werte nach Fikentscher von 30 bis 50, bevorzugt 35 bis 45 auf. Sie sind in für die Kosmetik üblichen Alkoholen wie Ethanol oder Isopropanol oder deren Gemischen mit Wasser klar löslich und gelfrei. Besonders vorteilhaft ist, daß sich auch höhere K-Werte gezielt einstellen lassen und die entstehenden Copolymerisate gute Filmbildungseigenschaften aufweisen.

Die erfindungsgemäßen Copolymerisate eignen sich zur Verwendung als Filmbildner in haarkosmetischen Zubereitungen wie Haarsprays, Haarfestigern oder Styling Cremes.

Beispiele

Die Trübung der wäßrigen Copolymerisat-Lösungen wurde durch nephelometrische Trübungsmessung bestimmt (modifizierte Methode nach DIN 38 404). Bei dieser Meßmethode wird photometrisch die Streuung von Licht nach Durchstrahlen der Meßlösung ermittelt, wobei diese Streuung durch die Wechselwirkung zwischen den Lichtstrahlen und den Partikeln oder Tröpfchen in der Lösung, deren Anzahl und Größe den Grad der Trübung ausmachen, bestimmt wird. Als Meßgröße dient die nephelometrische Trübungseinheit (NTU), welche bei 25°C und in 10-gew.-%iger wäßriger Lösung gemessen und durch Eichung auf der Basis von Formazin als künstlichem Trübungsmittel festgelegt wird. Je höher der NTU-Wert, desto trüber ist die Lösung.

Die Restgehalte an N-Vinylpyrrolidon und Vinylacetat in den Lösungen wurden durch gaschromatische Analyse (Nachweisgrenze 50 ppm) oder flüssigkeitschromatische Analyse (Nachweisgrenze 1 ppm) bestimmt.

Die in den Beispielen 1 bis 3 hergestellten Polymere sind völlig gelfrei, d.h. beim Ausstreichen der Polymerlösung zu einem Film waren keine Gelkörper zu erkennen.

Die K-Werte wurden in 1,0 gew.-%iger ethanolischer Lösung bei 25°C gemessen.

Zur Polymerisation wurde ein 5 1-Laborrührkessel der durch Spülen mit Stickstoff von Sauerstoffspuren weitestgehend befreit wurde.

Beispiel 1

Copolymerisat aus 30 Gew.-% N-Vinylpyrrolidon und 70 Gew.-% Vinylacetat in ethanolischer Lösung

Es wurden zunächst die folgenden Lösungen vorbereitet:

1. Lösung 1, bestehend aus 450 g N-Vinylpyrrolidon, 1050 g Vinylacetat und 870 g Ethanol.

2. Starterzulauf, bestehend aus 3 g t-Butylperoxypivalat gelöst in 100 g Ethanol.

3. Vorlage, bestehend aus 200 g Lösung 1 und 10 g Starterzulauf.

4. Monomerzulauf, bestehend aus 2120 g Lösung 1 und 4 g Divinylethylenharnstoff.

Die Vorlage wurde in den Laborrührkessel gegeben und bei einem Stickstoffdruck von 1,5 bar auf 70°C Innentemperatur erwärmt. Nach Anspringen der Polymerisation, erkennbar an Viskositätserhöhung und exothermer Reaktion wurde gleichzeitig der Monomerzulauf und der Starterzulauf gestartet. Der Starterzulauf wurde mit konstanter Geschwindigkeit in 8 Stunden zugefahren, der Monomerzulauf in 7 Stunden. Zur Restmonomereliminierung wurde die Innentemperatur von 70°C noch eine weitere Stunde gehalten, dann wurde eine Lösung aus 9 g 2,5-Dimethyl-2,5-di(tert.-Butylperoxy)hexan in 580 g Ethanol zudosiert und der geschlossene Kessel für 8 Stunden auf 130°C erwärmt.

Die erhaltene klare, farblose und viskose Lösung wies einen Feststoffgehalt von 50,0 % auf, der K-Wert des Produktes (gemessen 1 %ig in Ethanol) lag bei 43,4, beim Verdünnen der Lösung mit Ethanol auf 5 % Feststoffgehalt wurde eine Lösung mit NTU-Wert von 2,8 erhalten. Die Restmonomerwerte lagen bei Werten von kleiner als 50 ppm, sowohl für N-Vinylpyrrolidon, als auch für Vinylacetat.

Vergleichsbeispiel 1

Copolymerisat aus 30 Gew.-% N-Vinylpyrrolidon und 70 Gew.-% Vinylacetat in ethanolischer Lösung mit normalem K-Wert

Es wurden zunächst die folgenden Lösungen vorbereitet:

1. Monomerzulauf, bestehend aus 450 g N-Vinylpyrrolidon, 1050 g Vinylacetat und 870 g Ethanol.

2. Starterzulauf, bestehend aus 3 g t-Butylperoxypivalat gelöst in 100 g Ethanol.

Als Vorlage wurden 200 g Monomerzulauf und 10 g Starterzulauf in den Laborrührkessel gegeben, ein Stickstoffdruck von 1,5 bar eingestellt und auf 70°C Innentemperatur erwärmt. Das weitere Vorgehen erfolgte analog zu Beispiel 1.

Die erhaltene klare, farblose und viskose Lösung wies einen Feststoffgehalt von 51,5 % auf, der K-Wert des Produktes (gemessen 1 %ig in Ethanol) lag bei 28,7, beim Verdünnen der Lösung mit Ethanol auf 5 % Feststoffgehalt wurde eine Lösung mit NTU- Wert von 1,5 erhalten. Die Restmonomerwerte lagen bei Werten von kleiner als 50 ppm, sowohl für N-Vinylpyrrolidon, als auch für Vinylacetat.

Beispiel 2

Copolymerisat aus 60 Gew.-% N-Vinylpyrrolidon und 40 Gew.-% Vinylacetat in isopropanolischer Lösung mit erhöhtem K-Wert

Es wurden zunächst die folgenden Lösungen vorbereitet:

1. Lösung 1 bestehend aus 750 g N-Vinylpyrrolidon' 600 g Vinylacetat und 800 g Isopropanol.

2. Starterzulauf, bestehend aus 4 g t-Butylperoxypivalat gelöst in 100 g Ethanol.

3. Vorlage, bestehend aus 200 g Lösung 1 und 10 g Starterzulauf.

4. Monomerzulauf, bestehend aus 1950 g Lösung 1 und 3 g Divinylethylenharnstoff.

Die Vorlage wurde in den Laborrührkessel gegeben, ein Stickstoffdruck von 1,5 bar eingestellt und auf 70°C Innentemperatur erwärmt. Nach Anspringen der Polymerisation, erkennbar an Viskositätserhöhung und exothermer Reaktion wurde gleichzeitig der Monomerzulauf und der Starterzulauf gestartet. Der Starterzulauf wurde mit konstanter Geschwindigkeit in 8 Stunden zugefahren, der Monomerzulauf in 6 Stunden. Unmittelbar im Anschluß an den Monomerzulauf wurden weitere 150 g N-Vinylpyrrolidon innerhalb von zwei Stunden zudosiert.

Nach Starterzulauf Ende wurden 250 ml Isopropanol abdestilliert. Zur Restmonomereliminierung wurde eine Lösung aus 8 g t-Butylperoxypivalat in 150 g Isopropanol bei 70°C Innentemperatur innerhalb 8 Stunden zudosiert und die Temperatur von 70°C noch weitere 4 Stunden gehalten. Nach Zugabe von 750 g Wasser wurde das Isopropanol durch Wasserdampfdestillation ausgetrieben und die mit Wasser auf 20 % Feststoffgehalt eingestellte Lösung sprühgetrocknet.

Erhalten wurde ein leichtes, weißes Pulver. Der K-Wert des Produktes (gemessen 1 %ig in Ethanol) lag bei 37,8 (bei einem zweiten Versuch 39,3). In wäßriger Lösung (5 % Feststoffgehalt) wurde ein NTU-Wert von 2,1 gemessen. Die Restmonomerwerte lagen bei Werten von 150 ppm für N-Vinylpyrrolidon und kleiner als 50 ppm für Vinylacetat.

Vergleichsbeispiel 2

Copolymerisat aus 60 Gew. -% N-Vinylpyrrolidon und 40 Gew.-% Vinylacetat in isopropanolischer Lösung mit normalem K-Wert

Es wurden zunächst die folgenden Lösungen vorbereitet:

1. Monomerzulauf, bestehend aus 750 g N-Vinylpyrrolidon, 600 g Vinylacetat und 800 g Isopropanol.

2. Starterzulauf, bestehend aus 4 g t-Butylperoxypivalat gelöst in 100 g Ethanol.

Als Vorlage wurden 200 g Monomerzulauf und 10 g Starterzulauf in den Laborrührrkessel gegeben, ein Stickstoffdruck von 1,5 bar eingestellt und auf 70°C Innentemperatur erwärmt. Das weitere Vorgehen erfolgte analog zu Beispiel 2.

Erhalten wurde ein leichtes, weißes Pulver. Der K-Wert des Produktes (gemessen 1 %ig in Ethanol) lag bei 31,7. In wäßriger Lösung (5 % Feststoffgehalt) wurde ein NTU-Wert von 1,9 gemessen. Die Restmonomerwerte lagen bei Werten von 150 ppm für N-Vinylpyrrolidon und kleiner als 50 ppm für Vinylacetat.

Beispiel 3

Copolymerisat aus 50 Gew.-% N-Vinylpyrrolidon und 50 Gew.-% t-Butylacrylat in ethanolischer Lösung mit erhöhtem K-Wert

Es wurden zunächst die folgenden Lösungen vorbereitet:

1. Monomerzulauf, bestehend aus 675 g N-Vinylpyrrolidon, 750 g t-Butylacrylat, 5 g 1,4-Butandioldimethacrylat und 500 g Ethanol.

2. Starterzulauf, bestehend aus 4 g 2,2-Azobis (2-methyl-butyronitril) gelöst in 150 g Ethanol.

Die Vorlage, bestehend aus 75 g N-Vinylpyrrolidon, 75 g Ethanol und 15 g Starterzulauf, wurde in den Laborrührkessel gegeben, ein Stickstoffdruck von 1,5 bar eingestellt und auf 80°C Innentemperatur erwärmt. Nach Anspringen der Polymerisation, erkennbar an Viskositätserhöhung und exothermer Reaktion wurde gleichzeitig der Monomerzulauf und der Starterzulauf gestartet. Der Starterzulauf wurde mit konstanter Geschwindigkeit in 8 Stunden zugefahren, der Monomerzulauf in 6 Stunden. Zur Restmonomereliminierung wurde die Innentemperatur von 80°C noch eine weitere Stunde gehalten, dann wird eine Lösung aus 9 g Di-tert.-Butylperoxid in 825 g Ethanol zudosiert und der geschlossene Kessel für 8 Stunden auf 140°C erwärmt.

Die erhaltene klare, farblose und viskose Lösung wies einen Feststoffgehalt von 51,3 % auf, der K-Wert des Produktes (gemessen 1 %ig in Ethanol) lag bei 38,3, beim Verdünnen der Lösung mit Ethanol auf 5 % Feststoffgehalt wurde eine Lösung mit NTU- Wert von 1,0 erhalten. Die Restmonomerwerte lagen bei Werten von kleiner als 50 ppm, sowohl für N-Vinylpyrrolidon, als auch für t-Butylacrylat.

Vergleichsbeispiel 3

Copolymerisat aus 50 Gew. -% N-Vinylpyrrolidon und 50 Gew.-% t-Butylacrylat in ethanolischer Lösung mit normalem K-Wert

Es wurden zunächst die folgenden Lösungen vorbereitet:

1. Monomerzulauf; bestehend aus 675 g N-Vinylpyrrolidon, 750 g t-Butylacrylat und 500 g Ethanol.

2. Starterzulauf, bestehend aus 4 g 2,2-Azobis (2-methyl-butyronitril) gelöst in 150 g Ethanol.

Die Vorlage, bestehend aus 75 g N-Vinylpyrrolidon, 75 g Ethanol und 15 g Starterzulauf, wurde in den Laborrührkessel gegeben, ein Stickstoffdruck von 1,5 bar eingestellt und auf 80°C Innentemperatur erwärmt. Das weitere Vorgehen erfolgte analog zu Beispiel 3.

Die erhaltene klare, farblose und viskose Lösung wies einen Feststoffgehalt von 50,6 % auf, der K-Wert des Produktes (gemessen 1 %ig in Ethanol) lag bei 30,7, beim Verdünnen der Lösung mit Ethanol auf 5 % Feststoffgehalt wurde eine Lösung mit NTU- Wert von 0,9 erhalten. Die Restmonomerwerte lagen bei Werten von kleiner als 50 ppm, sowohl für N-Vinylpyrrolidon, als auch für t-Butylacrylat.

## Patentansprüche

1. Copolymerisate mit K-Werten von 30 bis 50 gemessen in 1 gew.-%iger ethanolischer Lösung bei 25°C, erhältlich durch radikalische Lösungspolymerisation von

    A) 15 bis 84,99 Gew.-% mindestens eines Monomeren aus der Gruppe N-Vinylpyrrolidon, N-Vinylcaprolactam und N-Vinylimidazol,

    B) 15 bis 84,99 Gew.-% weiterer radikalisch copolymerisierbarer monoolefinisch ungesättigter Monomeren und

    C) 0,01 bis 2 Gew.-% mindestens eines radikalisch copolymerisierbaren Monomeren mit mindestens zwei nicht konjugierten olefinischen Doppelbindungen

    in alkoholischer Lösung.

2. Copolymerisate nach Anspruch 1, enthaltend als Monomere A) N-Vinylpyrrolidon und/oder N-Vinylcaprolactam.

3. Copolymerisate nach Anspruch 1 oder 2, enthaltend als Monomere B) mindestens ein Monomer ausgewählt aus der Gruppe der Vinylester von gesättigten $C_2$-$C_{24}$-Monocarbonsäuren, der $C_1$-$C_{10}$-Alkylester von Acrylsäure oder Methacrylsäure, N, N-Dimethylaminoethylester der Methacrylsäure und dem Natriumsalz der Acrylamido-2-methyl-propan-sulfonsäure.

4. Verfahren zur Herstellung von Copolymerisaten gemäß den Ansprüchen 1 bis 3, dadurch gekennzeichnet, daß man eine Monomerenmischung aus

    A) 15 bis 84,99 Gew.-% mindestens eines Monomeren aus der Gruppe N-Vinylpyrrolidon, N-Vinylcaprolactam und N-Vinylimidazol,

    B) 15 bis 84,99 Gew.-% weiterer monoolefinisch ungesättigter Monomeren, und

    C) 0,01 bis 2 Gew.-% radikalisch polymerisierbarer Monomeren mit mindestens zwei nicht konjugierten olefinischen Doppelbindungen,

durch Lösungspolymerisation in alkoholischer Lösung in Gegenwart radikalbildender Verbindungen polymerisiert.

5. Verwendung von Copolymerisaten gemäß den Ansprüchen 1 bis 3 als Filmbildner.

6. Verwendung nach Anspruch 5, dadurch gekennzeichnet, daß der Filmbildner in haarkosmetischen Zubereitungen eingesetzt wird.

7. Haarkosmetische Zubereitungen, enthaltend als Filmbildner Copolymerisate gemäß den Ansprüchen 1 bis 3.

## Claims

1. A copolymer having a K value of from 30 to 50, measured in 1 % strength by weight ethanolic solution at 25°C, obtainable by free radical solution polymerization of

    A) from 15 to 84.99 % by weight of at least one monomer selected from the group consisting of N-vinylpyrrolidone, N-vinylcaprolactam and N-vinylimidazole,

    B) from 15 to 84.99 % by weight of further monoolefinically unsaturated monomers capable of free radical copolymerization and

    C) from 0.01 to 2 % by weight of at least one monomer capable of free radical copolymerization and having at least two nonconjugated olefinic double bonds

    in alcoholic solution.

2. A copolymer as claimed in claim 1, containing N-vinylpyrrolidone or N-vinylcaprolactam as monomer A).

3. A copolymer as claimed in claim 1 or 2, containing, as monomer B), at least one monomer selected from the group consisting of the vinyl esters of saturated $C_2$-$C_{24}$-monocarboxylic acids, the $C_1$-$C_{10}$-alkyl esters of acrylic acid or methacrylic acid, N,N-dimethylaminoethyl esters of methacrylic acid and the sodium salt of acrylamido-2-methylpropanesulfonic acid.

4. A process for the preparation of a copolymer as claimed in any of claims 1 to 3, wherein a monomer mixture of

    A) from 15 to 84.99 % by weight of at least one monomer selected from the group consisting of n-vinylpyrrolidone, N-vinylcaprolactam and N-vinylimidazole,

B) from 15 to 84.99 % by weight of further monoolefinically unsaturated monomers and

C) from 0.01 to 2 % by weight of monomers capable of free radical polymerization and having at least two nonconjugated olefinic double bonds

is polymerized by solution polymerization in alcoholic solution in the presence of a compound which forms free radicals.

5. Use of a copolymer as claimed in any of claims 1 to 3 as a film former.

6. Use as claimed in claim 5, wherein the film former is used in cosmetic hair formulations.

7. A cosmetic hair formulation containing a copolymer as claimed in any of claims 1 to 3 as a film former.

**Revendications**

1. Copolymères, ayant des indices K de 30 à 50 (mesurés sur une solution éthanolique à 1 % en poids à 25°C) obtenus par polymérisation radicalaire en solution de

A) 15 à 84,99 % en poids d'au moins un monomère du groupe de la N-vinylpyrrolidone, du N-vinylcaprolactame et du N-vinylimidazole,
B) 15 à 84,99 % en poids d'autres monomères à insaturation mono-oléfique aptes à la copolymérisation radicalaire et
C) 0,01 à 2 % en poids d'au moins un monomère à au moins deux doubles liaisons oléfiniques non conjuguées, apte à la copolymérisation radicalaire,

en solution alcoolique.

2. Copolymères selon la revendication 1, contenant, en tant que monomères A), la N-vinylpyrrolidone et/ou le N-vinylcaprolactame.

3. Copolymères selon la revendication 1 ou 2, contenant, en tant que monomère B), au moins un monomère choisi parmi les esters vinyliques d'acides monocarboxyliques saturés en C2-C24, les esters alkyliques en C1-C10 de l'acide acrylique ou de l'acide méthacrylique, l'ester N,N-diméthylaminoéthylique de l'acide méthacrylique et le sel de sodium de l'acide acrylamino-2-méthylpropane-sulfonique.

4. Procédé de préparation des copolymères selon les revendications 1 à 3, caractérisé par le fait que l'on polymérise un mélange de monomères consistant en

A) 15 à 84,99 % en poids d'au moins un monomère du groupe de la N-vinylpyrrolidone, du N-vinylcaprolactame et du N-vinylimidazole,
B) 15 à 84,99 % en poids d'autres monomères à insaturation mono-oléfinique et
C) 0,01 à 2 % en poids de monomères à au moins deux doubles liaisons oléfiniques non conjuguées, aptes à la polymérisation radicalaire,

par polymérisation en solution, en solution alcoolique, en présence d'inducteurs radicalaires.

5. Utilisation des copolymères selon les revendications 1 à 3 en tant que produits filmogènes.

6. Utilisation selon la revendication 5, caractérisée par le fait que les agents filmogènes sont utilisés dans des produits cosmétiques pour la chevelure.

7. Compositions cosmétiques pour la chevelure, contenant, en tant qu'agents filmogènes, des copolymères selon les revendications 1 à 3.